# EUROPÄISCHE PATENTSCHRIFT

(11) **EP 2 057 292 B1**
(45) Veröffentlichungstag und Bekanntmachung des Hinweises auf die Patenterteilung: **25.07.2018**
(21) Anmeldenummer: 07802529.3
(22) Anmeldetag: 08.08.2007
(51) Int. Cl.: C14C 3/18, C14C 9/02, C07D 251/00, C07D 251/66

(54) **MELAMIN-VERBINDUNGEN UND IHRE VERWENDUNG ZUR HERSTELLUNG VON LEDER**
MELAMINE COMPOUNDS AND THEIR USE FOR PRODUCING LEATHER
COMPOSÉS DE MÉLAMINE ET LEUR UTILISATION POUR LA FABRICATION DE CUIR

(30) Priorität: 23.08.2006 EP 06119375; 17.10.2006 EP 06122437
(43) Veröffentlichungstag der Anmeldung: 13.05.2009
(73) Patentinhaber: Stahl International B.V., 5145 PE Waalwijk (NL)
(72) Erfinder: HÜFFER, Stephan, 67063 Ludwigshafen (DE); GARNIER, Sebastien, 69469 Weinheim (DE); REESE, Oliver, 49448 Lemförde (DE); SCHERR, Günter, 67065 Ludwigshafen (DE); KIESOW, Harald, 67067 Ludwigshafen (DE)
(74) Vertreter: V.O.
(86) Internationale Anmeldenummer: PCT/EP2007/058209
(87) Internationale Veröffentlichungsnummer: WO 2008/022918

(56) Entgegenhaltungen:
- DE-A1- 2 118 868
- DE-A1- 10 143 984
- GB-A- 599 261
- US-A- 3 533 977
- DATABASE WPI Week 199331 Derwent Publications Ltd., London, GB; AN 1993-247938 XP002459201 & RO 103 351 A (INST CERC CHIM BUCURESTI) 25. Juni 1992 (1992-06-25)

## Beschreibung

Die vorliegende Erfindung betrifft ein Verfahren zur Herstellung von Leder unter Verwendung von einem oder mehreren Umsetzungsprodukten von
(a) Melamin
(b) mit mindestens einer Verbindung der allgemeinen Formel I 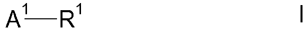
   wobei R¹ gewählt wird aus Kohlenwasserstoffresten mit 10 bis 5000 C-Atomen, unverzweigt oder verzweigt, gesättigt oder mit einer bis zu drei C-C-Doppelbindungen,
   und A¹ aus mit Aminen zur Reaktion befähigten Gruppen. Weiterhin betrifft die vorliegende Erfindung Verbindungen, unter deren Verwendung sich das vorstehend genannte Verfahren besonders gut durchführen lässt. Weiterhin betrifft die vorliegende Erfindung Leder, die nach dem erfindungsgemäßen Verfahren hergestellt sind, sowie deren Verwendung.

Leder finden bei der Herstellung von zahlreichen Gegenständen Verwendung, beispielsweise von Autoinnenteilen, Möbeln und von Bekleidungsmitteln wie Jacken, Schuhen und Mänteln. Dabei sind hochwertige Bekleidungsstücke besonders interessant. Zur Reinigung und Pflege beispielsweise nach starker Verschmutzung ist es wünschenswert, derartige Bekleidungsstücke zu waschen. Man beobachtet jedoch, dass derartige Bekleidungsstücke nach einigen Wäschen und in manchen Fällen schon nach einer einzigen Wäsche eine starke Qualitätseinbuße zeigen. Bei der Wäsche werden in der Regel die weichmachenden Fettungsmittel-Komponenten zumindest partiell extrahiert, was sich u.a. in einer Versprödung des Leders, in einem Verlust an Farbtiefe insbesondere in einer Vergrauung von schwarzen Leder sowie allgemein durch einen unangenehmen Griff äußert. So lässt die Farbe nach, und der Griff wird unangenehm. In einzelnen Fällen muss man sogar Rissbildung feststellen.

Es gibt zahlreiche Versuche, das Problem zu lösen, indem man eine chemische, beispielsweise eine kovalente Fixierung der Farbstoffe durchführt, siehe beispielsweise WO 05/40490. Dennoch beobachtet man, dass die Färbung beispielsweise von aus Leder hergestellten Reithosen in der Brillanz nach einigen Wäschen nachlässt und der Griff weniger angenehm ist.

RO103351 offenbart ein Fettungsmittel für natürliches Leder und deren Herstellmethoden.

Es bestand also die Aufgabe, ein Verfahren zur Herstellung von Leder bereit zu stellen, dass die vorstehend genannten Nachteile vermeidet und Leder mit guter Beständigkeit der Färbung und weiteren guten Gebrauchseigenschaften, insbesondere gutem Griff liefert.

Dementsprechend wurde das eingangs definierte Verfahren gefunden.

Zur Ausübung des eingangs definierten Verfahrens geht man aus von nach konventionellen Methoden vorbehandelten Häuten von Tieren wie beispielsweise Rindern, Schweinen, Ziegen oder Hirschen, den sogenannten Blößen. Dabei ist es für das erfindungsgemäße Verfahren nicht wesentlich, ob die Tiere beispielsweise durch Schlachten getötet wurden oder aber an natürlichen Ursachen verendet sind. Zu den konventionellen Methoden der Vorbehandlung gehören das beispielsweise das Äschern, Entkälken, Beizen und Pickeln sowie mechanische Arbeitsschritte, beispielsweise die Entfleischung der Häute.

Zur Ausübung des erfindungsgemäßen Verfahrens verwendet man ein oder mehrere Umsetzungsprodukte, erhältlich durch Umsetzung von
(a) Melamin, mit
(b) mindestens einer Verbindung der allgemeinen Formel I 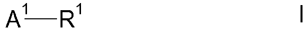
   wobei R¹ gewählt wird aus Kohlenwasserstoffresten mit 10 bis 5000 C-Atomen, unverzweigt oder verzweigt, gesättigt oder mit einer bis zu drei C-C-Doppelbindungen,
   und A¹ aus mit Aminen zur Reaktion befähigten Gruppen. Derartige Umsetzungsprodukte von Melamin (a) mit Verbindung der allgemeinen Formel I (b) werden im Folgenden auch als erfindungsgemäß eingesetzte Umsetzungsprodukte bezeichnet.

Melamin (a) wird umgesetzt mit (b) mindestens einer Verbindung der allgemeinen Formel I

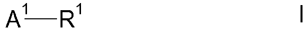

Dabei sind die Variablen wie folgt definiert:
A¹ ist eine zur Reaktion mit Amin befähigte Gruppe, d. h. eine Gruppe, die mit einer organischen Aminogruppe im Sinne einer Additionsreaktion oder vorzugsweise Substitutionsreaktion reagieren kann. Beispiele für Gruppen, die unter Addition mit Amin reagieren können, sind Isocyanatgruppen und zu Michael-Additionen befähigte Gruppen, beispielsweise -CO-CH=CH₂-Gruppen.

Bevorzugt als A¹ sind solche Gruppen, die mit Aminogruppen im Sinne einer Sustitutionsreaktion reagieren können, insbesondere Carbonylgruppen und Carboxylgruppen. Beispiele sind Säurechloridgruppen (CO-Cl), Chlorameisensäureestergruppen (O-CO-Cl), C₁-C₄-Alkylestergruppen, insbesondere Ethyl- und Methylestergruppen, und Carbonsäureanhydridgruppen, insbesondere Bernsteinsäureanhydridgruppen.

R¹ ist gewählt aus Kohlenwasserstoffresten mit 10 bis 5000 C-Atomen, bevorzugt 15 bis 2500 C-Atomen, besonders bevorzugt bis 500 C-Atomen, linear oder vorzugsweise verzweigt, gesättigt oder mit einer bis zu 3 C-C-Doppelbindungen, die cis- oder trans-Konfiguration aufweisen können oder als Methyliden-Gruppe vorliegen können. R¹ weist vorzugsweise keine Heteroatome auf.

Bevorzugte Beispiele für R¹ sind n-Alkylreste mit 10 bis 40 C-Atomen, bevorzugt 12 bis 20 C-Atomen, beispielsweise n-C₁₂H₂₅, n-C₁₄H₂₉, n-C₁₆H₃₃, n-C₁₈H₃₇ und n-C₂₀H₄₁.

Besonders bevorzugte Beispiele für R¹ sind Polyisobutenylreste, insbesondere solche der Formel 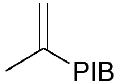 wobei PIB einen Rest bezeichnet, der von Polyisobuten abgeleitet ist, beispielsweise

(CH₃)₃C-[CH₂-C(CH₃)₂]ₙ-CH₂-

wobei n eine Zahl im Bereich von 1 bis etwa 5000, bevorzugt 2 bis etwa 2500, besonders bevorzugt bis 500 sein kann. Dabei handelt es sich bei n in der Regel um Mittelwerte (Zahlenmittel), und n kann auch eine nicht-ganze Zahl sein.

In einer Ausführungsform der vorliegenden Erfindung sind bis zu 20 mol-%, bevorzugt 1 bis 10 mol-% der [CH₂-C(CH₃)₂]-Einheiten im PIB durch ein oder mehrere unverzweigte oder vorzugsweise verzweigte C₄-C₁₀-Olefine ersetzt. Beispielhaft seien genannt: 1-Penten, 2-Methylbuten-1, 1-Hexen, 2-Methylpenten-1, 2-Methylhexen-1, 2,4-Dimethyl-1-hexen, Diisobuten (Gemisch aus 2,4,4-Trimethyl-1-penten und 2,4,4-Trimethyl-2-penten), 2-Ethylpenten-1, 2-Ethylhexen-1 und 2-Propylhepten-1, 1-Okten, 1-Decen und 1-Dodecen sowie Styrol.

In einer anderen Ausführungsform der vorliegenden Erfindung handelt es sich bei PIB um Reste, die außer Isobuten keine weiteren Olefine einpolymerisiert enthalten.

Viele Verbindungen der allgemeinen Formel I sind bekannt, auf die Herstellung einiger Beispiele und die Herstellung ihrer Homologen wird unten näher eingegangen.

In einer Ausführungsform der vorliegenden Erfindung übt man das erfindungsgemäße Verfahren als Gerbverfahren aus, im Folgenden auch als erfindungsgemäßes Gerbverfahren bezeichnet, bevorzugt als Nachgerbverfahren, im Folgenden auch als erfindungsgemäßes Nachgerbverfahren bezeichnet.

Das erfindungsgemäße Gerbverfahren übt man im Allgemeinen so aus, dass man ein oder mehrere erfindungsgemäß eingesetzte Umsetzungsprodukte in einer Portion oder in mehreren Portionen unmittelbar vor oder aber während des Gerbungsschrittes zusetzt. Das erfindungsgemäße Gerbverfahren wird vorzugsweise bei einem pH-Wert von 2,5 bis 4 durchgeführt, wobei man häufig beobachtet, dass der pH-Wert während der Durchführung des erfindungsgemäßen Gerbverfahrens um etwa 0,3 bis drei Einheiten ansteigt.

Das erfindungsgemäße Gerbverfahren führt man im Allgemeinen bei Temperaturen von 10 bis 45°C, bevorzugt bei 20 bis 30°C durch. Bewährt hat sich eine Dauer von 10 Minuten bis 12 Stunden, bevorzugt sind eine bis drei Stunden. Das erfindungsgemäße Gerbverfahren kann man in beliebigen gerbereiüblichen Gefäßen durchführen, beispielsweise durch Walken in Fässern oder in gedrehten Trommeln.

In einer Ausführungsform der vorliegenden Erfindung setzt man insgesamt 0,01 bis 10 Gew.-% erfindungsgemäß eingesetztes Umsetzungsprodukt, bezogen auf das Falzgewicht ein, bevorzugt 0,5 bis 3 Gew.-%.

In einer Variante des erfindungsgemäßen Gerbverfahrens setzt man erfindungsgemäß eingesetztes Umsetzungsprodukt zusammen mit einem oder mehreren herkömmlichen Gerbstoffen ein, beispielsweise mit Chromgerbstoffen, mineralischen Gerbstoffen, Syntanen, Polymergerbstoffen oder vegetabilen Gerbstoffen, wie sie beispielsweise beschrieben sind in Ullmann's Encyclopedia of Industrial Chemistry, Band A15, Seite 259 bis 282 und insbesondere Seite 268 ff., 5. Auflage, (1990), Verlag Chemie Weinheim. Das Gewichtsverhältnis Produkt, erhalten durch Umsetzung von Triamin (a) bzw. höherem Amin (a) mit Verbindung der allgemeinen Formel I (b) : herkömmlicher Gerbstoff bzw. Summe der herkömmlichen Gerbstoffe beträgt zweckmäßig von 0,01 : 1 bis 100 : 1. In einer vorteilhaften Variante des erfindungsgemäßen Verfahrens setzt man nur wenige ppm herkömmliches Gerbmittel oben beschriebenem Produkt, erhalten durch Umsetzung von Melamin (a) mit Verbindung der allgemeinen Formel I (b), zu.

In einer Variante des erfindungsgemäßen Gerbverfahrens setzt man erfindungsgemäß eingesetztes Umsetzungsprodukt zusammen mit einem oder mehreren Fettungsmitteln bzw. oleophilen Komponenten ein.

In einer Variante des erfindungsgemäßen Gerbverfahrens setzt man erfindungsgemäß eingesetztes Umsetzungsprodukt in einer Portion oder in mehreren Portionen vor oder während des Vorgerbens zu. Auch ein Zusatz im Pickel ist denkbar.

Zur Durchführung des erfindungsgemäßen Nachgerbverfahrens geht man aus von konventionell, d.h. beispielsweise mit Chromgerbstoffen, mineralischen Gerbstoffen, Polymergerbstoffen, Aldehyden, Syntanen oder Harzgerbstoffen gegerbten Halbzeugen oder erfindungsgemäß wie oben beschrieben hergestellten Halbzeugen. Zur Durchführung des erfindungsgemäßen Nachgerbung lässt man mindestens ein erfin

dungsgemäß eingesetztes Umsetzungsprodukt auf Halbzeuge einwirken, d.h., man behandelt mit mindestens einem erfindungsgemäß eingesetzten Umsetzungsprodukt.

Das erfindungsgemäße Nachgerbverfahren kann man unter ansonsten üblichen Bedingungen durchführen. Man wählt zweckmäßig einen oder mehrere, d.h. 2 bis 6 Einwirkschritte und kann zwischen den Einwirkschritten mit Wasser spülen. Die Temperatur bei den einzelnen Einwirkschritten beträgt jeweils von 5 bis 60°C, bevorzugt 20 bis 45°C. Man setzt zweckmäßig einen oder mehrere weitere, während der Nachgerbung üblicherweise verwendete Mittel ein, beispielsweise Fettlicker, Polymergerbstoffe und Fettungsmittel auf Acrylat- und/oder Methacrylatbasis, Nachgerbstoffe auf Basis von Vegetabilgerbstoffen, Füllstoffe, Lederfarbstoffe oder Emulgatoren.

In einer Ausführungsform der vorliegenden Erfindung behandelt man zusätzlich mit mindestens einer hydrophoben Verbindung, vorzugsweise gewählt aus Silikonen, nativen Fetten und bevorzugt Polyisobuten. Dabei werden unter Polyisobuten Homopolymere und Copolymere des Isobutens verstanden mit bis zu 20 mol-% Propylen, 1-Penten, 2-Methylbuten-1, 1-Hexen, 2-Methylpenten-1, 2-Methylhexen-1, 2,4-Dimethyl-1-hexen, Diisobuten (Gemisch aus 2,4,4-Trimethyl-1-penten und 2,4,4-Trimethyl-2-penten), 2-Ethylpenten-1, 2-Ethylhexen-1 und 2-Propylhepten-1, 1-Okten, 1-Decen und 1-Dodecen oder Vinylaromaten wie Styrol und α-Methylstyrol, C₁-C₄-Alkylstyrol wie beispielsweise 2-, 3- und 4-Methylstyrol sowie 4-tert.-Butylstyrol, ganz besonders bevorzugt sind Homopolymere von Isobuten. Polyisobuten im Sinne der vorliegenden Erfindung kann pro Molekül eine ethylenisch ungesättigte Gruppe aufweisen, die in Form einer Vinyl-, Vinyliden- oder Alkylvinylidengruppe vorliegen kann.

In einer Ausführungsform der vorliegenden Erfindung hat Polyisobuten ein mittleres Molekulargewicht Mₙ von bis zu 50.000 g/mol, bevorzugt 300 bis 25.000 g/mol, besonders bevorzugt 400 bis zu 10.000 g/mol, ganz besonders bevorzugt 500 bis zu 5000 g/mol und noch mehr bevorzugt bis zu 1200 g/mol, bestimmt beispielsweise durch Gelpermeationschromatographie (GPC).

In einer Ausführungsform der vorliegenden Erfindung weist Polyisbuten eine Polydispersität M_{w}/Mₙ im Bereich von 1,1 bis 10, bevorzugt bis 3 und besonders bevorzugt von 1,5 bis 2,0 auf.

In einer Ausführungsform weist Polyisobuten eine monomodale Molekulargewichtsverteilung auf. In einer anderen Ausführungsform der vorliegenden Erfindung weist Polyisobuten eine multimodale und insbesondere eine bimodale Molekulargewichtsverteilung auf mit einem Maximum von Mₙ im Bereich von 500 bis 1200 g/mol und einem lokalen Maximum von Mₙ im Bereich von 2000 bis 50.000 g/mol, besonders bevorzugt bis 10.000 g/mol.

Ein weiterer Gegenstand der vorliegenden Erfindung sind Leder, hergestellt nach dem erfindungsgemäßen Verfahren. Erfindungsgemäße Leder zeichnen sich durch gute Fülle, Weichheit und Intensität und Waschbeständigkeit der Färbung und weitere gute Gebrauchseigenschaften aus. Erfindungsgemäße Leder eignen sich beispielsweise zur Herstellung von Schuhen oder Autoinnenteilen wie z. B: Autositzen und insbesondere zur Herstellung von Bekleidungsstücken wie beispielsweise Gürteln, Jacken, Mänteln und Hosen, insbesondere Sporthosen wie beispielsweise Reithosen, weiterhin beispielsweise zur Herstellung von Möbeln und von Handtaschen.

Ein weiterer Gegenstand der vorliegenden Erfindung sind Verbindungen der Formel II 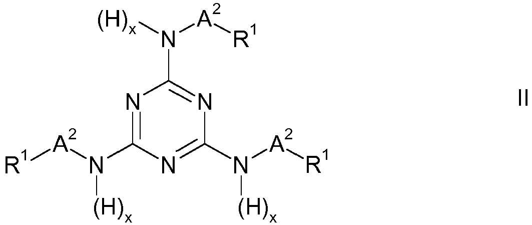 in denen die Variablen wie folgt definiert sind:
R¹ gleich oder verschieden und gewählt aus Kohlenwasserstoffresten mit 10 bis 5000 C-Atomen, unverzweigt oder verzweigt, gesättigt oder mit einer bis zu drei C-C-Doppelbindungen,
N-A² gleich oder verschieden und gewählt aus funktionellen Einheiten, die durch Reaktion von mit Amin zur Reaktion befähigten Gruppen A¹ bei der Reaktion mit Amin entstehen,
x gleich oder verschieden und jeweils gewählt aus Null oder eins, vorzugsweise jeweils gleich null.

R¹ ist gewählt aus Kohlenwasserstoffresten mit 10 bis 5000 C-Atomen, bevorzugt 15 bis 2500 C-Atomen, besonders bevorzugt bis 500 C-Atomen, linear oder vorzugsweise verzweigt, gesättigt oder mit einer bis zu 3 C-C-Doppelbindungen, die cis- oder trans-Konfiguration aufweisen können oder als Methyliden-Gruppe vorliegen können. R¹ weist vorzugsweise keine Heteroatome auf.

Bevorzugte Beispiele für R¹ sind n-Alkylreste mit 10 bis 40 C-Atomen, bevorzugt 12 bis 20 C-Atomen, beispielsweise n-C₁₂H₂₅, n-C₁₄H₂₉, n-C₁₆H₃₃, n-C₁₈H₃₇ und n-C₂₀H₄₁.

Besonders bevorzugte Beispiele für R¹ sind Polyisobutenylreste, insbesondere solche der Formel 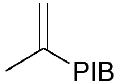 wobei PIB einen Rest bezeichnet, der von Polyisobuten abgeleitet ist, beispielsweise

(CH₃)₃C-[CH₂-C(CH₃)₂]ₙ-CH₂-

wobei n eine Zahl im Bereich von 1 bis etwa 5000, bevorzugt 2 bis etwa 2500, besonders bevorzugt bis 500 sein kann. Dabei handelt es sich bei n in der Regel um Mittelwerte (Zahlenmittel), und n kann auch eine nicht-ganze Zahl sein.

In einer Ausführungsform der vorliegenden Erfindung sind bis zu 20 mol-%, bevorzugt 1 bis 10 mol-% der [CH₂-C(CH₃)_{2]}-Einheiten im PIB durch ein oder mehrere unverzweigte oder vorzugsweise verzweigte C₄-C₁₀-Olefine ersetzt. Beispielhaft seien genannt: 1-Penten, 2-Methylbuten-1, 1-Hexen, 2-Methylpenten-1, 2-Methylhexen-1, 2,4-Dimethyl-1-hexen, Diisobuten (Gemisch aus 2,4,4-Trimethyl-1-penten und 2,4,4-Trimethyl-2-penten), 2-Ethylpenten-1, 2-Ethylhexen-1 und 2-Propylhepten-1, 1-Okten, 1-Decen und 1-Dodecen sowie Styrol.

In einer anderen Ausführungsform der vorliegenden Erfindung handelt es sich bei PIB um Reste, die außer Isobuten keine weiteren Olefine einpolymerisiert enthalten.

N-A² ist jeweils gleich oder verschieden und gewählt aus funktionellen Einheiten, die durch Reaktion von Amin mit zur Reaktion mit Amin befähigter Gruppe A¹ bei der Reaktion mit Amin entstehen. So ist für den Fall, dass A¹ eine Isocyanatgruppe ist, N-A² eine N-CO-NH-Gruppe. Für den Fall, dass A¹ eine Chlorkohlensäureestergruppe ist, ist N-A² eine N-CO-O-Gruppe. In den vorstehend genannten Fällen ist x vorzugsweise jeweils gleich eins.

Für den Fall, dass A¹ gewählt wird aus Säurechloridgruppen (CO-Cl) und C₁-C₄-Alkylestergruppen, insbesondere Ethyl- und Methylestergruppen, ist N-A² vorzugsweise eine Amidgruppe und x jeweils eins.

In einer Ausführungsform der vorliegenden Erfindung ist A¹ eine Bernsteinsäureanhydridgruppe, und N-A² ist eine Imidgruppe der Formel 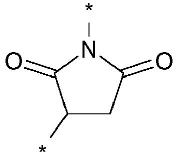 und x ist jeweils gleich null.

Ein weiterer Gegenstand der vorliegenden Erfindung sind Verbindungen der allgemeinen Formel III und bevorzugt III b, 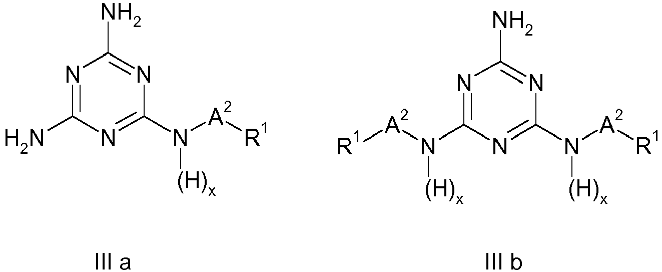 in denen die Variablen wie vorstehend definiert sind.

Erfindungsgemäße Verbindungen der Formel III a, bevorzugt III b und insbesondere II sind zur Durchführung des Verfahrens zur Herstellung von Leder besonders gut geeignet. Ein weiterer Gegenstand der vorliegenden Erfindung ist die Verwendung von erfindungsgemäßer Verbindung zur Herstellung von Leder, beispielsweise als Gerbmittel oder Nachgerbmittel.

Ein weiterer Gegenstand der vorliegenden Erfindung ist ein Verfahren zur Herstellung von erfindungsgemäßen Verbindungen, im Folgenden auch als erfindungsgemäßes Herstellverfahren bezeichnet. Zur Durchführung des erfindungsgemäßen Herstellverfahrens kann man so vorgehen, dass man Triamin oder höheres Amin, vorzugsweise Melamin mit mindestens einer Verbindung der allgemeinen Formel I umsetzt.

Verbindungen der allgemeinen Formel I und ihre Herstellung sind an sich bekannt. Besonders bevorzugte Verbindungen der allgemeinen Formel I, bei denen R¹ gewählt wird aus Kohlenwasserstoffresten der Formel 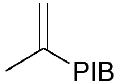 kann man durch Funktionalisierung von sogenanntem Reaktivem Polyisobuten, also Polyisobuten mit mindestens einer C-C-Doppelbindung, herstellen. Methoden zur Funktionalisierung von Polyisobuten sind an sich bekannt, beispielhaft seien erwähnt:
i) Umsetzung von Polyisobuten mit einer äquimolaren Menge an Peroxy-Verbindung, beispielsweise H₂O₂ oder einer Peroxycarbonsäure wie *meta-*Chlorperbenzoesäure, unter Erhalt eines epoxidierten Polyisobutens,
ii) Umsetzung von Polyisobuten mit einem Alken, das eine mit einer oder vorzugsweise mehreren elektronenziehenden Gruppen substituierte Doppelbindung aufweist (Enophil), in einer En-Reaktion, besonders bevorzugtes Enophil ist Maleinsäureanhydrid,
iii) Umsetzung von Polyisobuten mit Kohlenmonoxid und Wasserstoff in Gegenwart eines Hydroformylierungskatalysators unter Erhalt eines hydroformylierten Polyisobutens,
iv) Umsetzung von Polyisobuten mit einem Phosphorhalogenid oder einem Phosphoroxychlorid unter Erhalt eines mit Phosphongruppen funktionalisierten Polyisobutens.

In einer Ausführungsform der vorliegenden Erfindung setzt man Melamin mit einer Verbindung der allgemeinen Formel IV 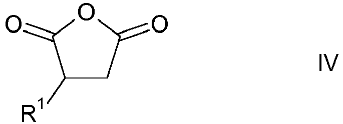 um, wobei R¹ wie vorstehend definiert ist.

Man kann das erfindungsgemäße Herstellverfahren bei Zimmertemperatur durchführen. Es ist jedoch bevorzugt, das erfindungsgemäße Herstellverfahren bei erhöhter Temperatur, beispielsweise bei 50 bis 200 °C, bevorzugt 150 bis 195 °C durchzuführen.

Man kann das erfindungsgemäße Herstellverfahren bei Normaldruck durchführen. In einer Ausführungsform der vorliegenden Erfindung führt man das erfindungsgemäße Herstellverfahren bei erhöhtem Druck, beispielsweise bei 1,1 bis 10 bar durch. In einer anderen Ausführungsform der vorliegenden Erfindung führt man das erfindungsgemäße Verfahren bei reduziertem Druck, beispielsweise bei 10 bis 750 mbar durch.

In einer Ausführungsform der vorliegenden Erfindung kann man Melamin und Verbindung der Formel I im stöchiometrischen Verhältnis einsetzen, bezogen auf Aminogruppen. In einer bevorzugten Ausführungsform der vorliegenden Erfindung setzt man einen Überschuss an Verbindung I ein, beispielsweise einen 1,1- bis 10-fachen Überschuss, bezogen auf Aminogruppen.

In einer Ausführungsform der vorliegenden Erfindung kann man das erfindungsgemäße Herstellverfahren unter Verwendung von einem Lösungsmittel durchführen. Geeignet sind insbesondere hochsiedende Lösungsmittel wie beispielsweise Xylole oder Ethylbenzol, weiterhin N,N-Dimethylformamid. In einer bevorzugten Ausführungsform der vorliegenden Erfindung führt man das erfindungsgemäße Herstellverfahren ohne Verwendung von Lösungsmitteln durch.

In einer Ausführungsform der vorliegenden Erfindung kann man erfindungsgemäße Verbindung nach erfolgter Umsetzung aufreinigen, beispielsweise durch Extraktion. In einer bevorzugten Ausführungsform der vorliegenden Erfindung unterlässt man jedoch Aufreinigungsschritte und verwendet das anfallende Rohprodukt für das erfindungsgemäße Verfahren.

In einer Ausführungsform der vorliegenden Erfindung ist erfindungsgemäße Verbindung bei der Verwendung zur Herstellung von Leder verunreinigt mit Verbindung der allgemeinen Formel I.

In einer Ausführungsform der vorliegenden Erfindung ist erfindungsgemäße Verbindung bei der Verwendung zur Herstellung von Leder verunreinigt mit hydrolysierter Verbindung der allgemeinen Formel I. So kann beispielsweise dann, wenn es sich bei der Verbindung der allgemeinen Formel I um PIBSA 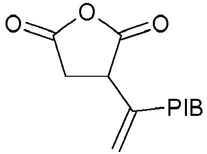 handelt, bei der Verwendung zur Herstellung von Leder als Verunreinigung eine Verbindung der Formel 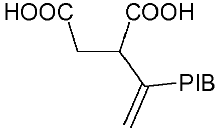 detektiert werden.

Insbesondere kann man Polyisobuten, reaktiv (mit C-C-Doppelbindung) oder nichtreaktiv (keine C-C-Doppelbindungen oder eine interne C-C-Doppelbindung pro Molekül) in vielen Fällen als Verunreinigung feststellen.

Insbesondere kann man weiterhin die korrespondierenden Verbindungen der Formel III a und bevorzugt III b als Verunreinigung in Verbindungen der Formel II finden. Umgekehrt kann man die korrespondierenden Verbindungen der allgemeinen Formeln III a und II als Verunreinigung in erfindungsgemäßen Verbindungen der allgemeinen Formel III b finden.

In einer Ausführungsform der vorliegenden Erfindung weisen erfindungsgemäße Verbindungen der Formel III a, bevorzugt III b und insbesondere II eine dynamische Viskosität im Bereich von 500 bis 500.000, bevorzugt 2.500 bis 250.000 mPa·s auf, bestimmt bei 23°C beispielsweise nach ISO-Norm 3219.

Ein weiterer Aspekt der vorliegenden Erfindung sind Gemische, enthaltend mindestens eine erfindungsgemäße Verbindung der Formel III a, bevorzugt III b und insbesondere II, wie vorstehend beschrieben, und Polyisobuten. Mit erfindungsgemäßen Gemischen lässt sich das erfindungsgemäße Verfahren zur Herstellung von Leder ebenfalls sehr gut durchführen. Ein weiterer Gegenstand der vorliegenden Erfindung ist somit die Verwendung von erfindungsgemäßen Gemischen als Hilfsmittel zur Lederherstellung. Ein weiterer Aspekt der vorliegenden Erfindung ist ein Verfahren zur Herstellung von Leder unter Verwendung von mindestens einem erfindungsgemäßen Gemisch. Das letztgenannte erfindungsgemäße Verfahren kann man insbesondere als Gerb- oder Nachgerbverfahren ausüben. Zu den Verfahrensbedingungen gilt prinzipiell das oben genannte.

In einer Ausführungsform der vorliegenden Erfindung liegen erfindungsgemäße Verbindung der Formel III a, bevorzugt III b und insbesondere II und Polyisobuten in einem Gewichtsverhältnis im Bereich von 20 zu 1 bis 5 zu 1 vor, bevorzugt 12 zu 1 bis 8 zu 1.

In einer Ausführungsform der vorliegenden Erfindung weisen erfindungsgemäße Gemische eine dynamische Viskosität im Bereich von 10.000 bis 500.000, bevorzugt 50.000 bis 250.000 mPa·s auf, bestimmt bei 23°C beispielsweise nach ISO-Norm 3219.

Ein weiterer Gegenstand der vorliegenden Erfindung sind wässrige Formulierungen, beispielsweise wässrige Lösungen, enthaltend mindestens eine erfindungsgemäße Verbindung der Formel III a, bevorzugt III b und insbesondere II. Erfindungsgemäße wässrige Formulierungen können gelblich oder auch bräunlich sein und einen Feststoffgehalt im Bereich von 1 bis 90 Gew.-%, bevorzugt 30 bis 75 Gew.-% aufweisen. Erfindungsgemäße wässrige Formulierungen sind zur Durchführung des erfindungsgemäßen Verfahrens zur Herstellung von Leder besonders gut geeignet und lassen sich gut dosieren. Ein weiterer Gegenstand der vorliegenden Erfindung ist die Verwendung von erfindungsgemäßer wässriger Formulierung zur Herstellung von Leder, beispielsweise als Gerbmittel oder Nachgerbmittel.

Die Erfindung wird durch Arbeitsbeispiele erläutert.

Dynamische Viskositäten wurden stets nach ISO-Norm 3219 - ehemalige DIN-Norm 53018 bestimmt.
I. Herstellung von erfindungsgemäßen Verbindungen und erfindungsgemäßen Gemischen

### I.1 Herstellung von erfindungsgemäßem Gemisch I.1

In einem Glaskolben mit Rührer wurden 50,0 g (0,039 mol) Polyisobutenylbernsteinsäureanhydrid mit einer Verseifungszahl von 87,5 mg KOH/g (Mᵣₑₐₖₜᵢᵥ = 1282 g/mol) mit 0,47 g (0,0037 Mol) Melamin versetzt und auf 170 °C erhitzt. Das entstehende Reaktionsgemisch wurde 4 Stunden bei 170°C gerührt und danach auf Zimmertemperatur abgekühlt. Man erhielt 41,1 g erfindungsgemäßes Gemisch I.1 als homogenes, durchsichtiges, dunkelbraunes, dickes und klebendes Kondensat. Die dynamische Viskosität betrug 158.000 mPa·s bei 23 °C. Charakteristische Peaks im IR-Spektrum: 1780 cm⁻¹ und 1865 cm⁻¹, 1780 cm⁻¹ und 1714 cm⁻¹.

### I.2 Herstellung von erfindungsgemäßem Gemisch I.2

50,0 g (0,039 mol) Polyisobutenylbernsteinsäureanhydrid analog zu Beispiel I.1 wurden mit 1,89 g (0,015 Mol) Melamin versetzt und auf 170 °C erhitzt. Das entstehende Reaktionsgemisch wurde 7 Stunden bei 170°C gerührt und danach auf Zimmertemperatur abgekühlt. Man erhielt 39,0 g erfindungsgemäßes Gemisch I.2 als dunkelbraunes, dickes und klebendes Kondensat. Die dynamische Viskosität betrug 184.000 mPa·s bei 23 °C. Charakteristische Peaks im IR-Spektrum: 1780 cm⁻¹ und 1712 cm⁻¹. Nebenpeaks: 1780 cm⁻¹ und 1865 cm⁻¹.

### I.3 Herstellung von erfindungsgemäßem Gemisch I.3

46,5 g (0,0347 mol) Polyisobutenylbernsteinsäureanhydrid mit einer Verseifungszahl von 155,8 (Mᵣₑₐₖₜᵢᵥ = 720 g/mol) wurden mit 0,82 g (0,0065 Mol) Melamin versetzt und auf 170 °C erhitzt. Das entstehende Reaktionsgemisch wurde 4 Stunden bei 170°C gerührt und danach auf Zimmertemperatur abgekühlt. Man erhielt 40,7 g erfindungsgemäßes Gemisch I.3 als homogenes, durchsichtiges, dunkelbraunes, dickes und klebendes Kondensat. Die dynamische Viskosität betrug 16.290 mPa·s bei 40 °C. Charakteristische Peaks im IR-Spektrum: 1780 cm⁻¹ und 1865 cm⁻¹, 1780 cm⁻¹ und 1714 cm⁻¹.

### I.4 Herstellung von erfindungsgemäßem Gemisch I.4

50,0 g (0,02 Mol) Polyisobutenylbernsteinsäureanhydrid mit einer Verseifungszahl von 45,6 (Mᵣₑₐₖₜᵢᵥ = 2460 g/mol) wurden mit 0,25 g (0,002 Mol) Melamin versetzt und auf 120 °C erhitzt. Das entstehende Reaktionsgemisch wurde 6 Stunden bei 120°C gerührt und danach auf Zimmertemperatur abgekühlt. Man erhielt 44,4 g erfindungsgemäßes Gemisch I.4 als homogenes, durchsichtiges, dunkelbraunes, dickes und klebendes Kondensat. Die dynamische Viskosität betrug 244.000 mPas bei 40 °C. Charakteristische Peaks im IR-Spektrum: 1780 cm⁻¹ und 1865 cm⁻¹, 1780 cm⁻¹ und 1713 cm⁻¹.

### I.5 Herstellung von erfindungsgemäßem Gemisch I.5

50,0 g (0,039 mol) Polyisobutenylbernsteinsäureanhydrid mit einer Verseifungszahl von 87,5 mg KOH/g (Mᵣₑₐₖₜᵢᵥ = 1282 g/mol) wurden mit 0,47 g (0,0037 mol) Melamin versetzt und auf 120°C erhitzt. Das entstehende Reaktionsgemisch wurde 4 Stunden bei 120°C gerührt und danach auf Zimmertemperatur abgekühlt. Man erhielt 41,1 g erfindungsgemäßes Gemisch I.5 als homogenes, durchsichtiges, hellbraunes, dickes und klebendes Kondensat. Die dynamische Viskosität betrug 32.590 mPa·s bei 40 °C. Charakteristische Peaks im IR-Spektrum: 1780 cm⁻¹ und 1865 cm⁻¹, 1780 cm⁻¹ und 1714 cm⁻¹.

### I.6 Herstellung von erfindungsgemäßem Gemisch I.6

In einer ersten Stufe wurde ein Anilinharzöl als Beispiel für ein höheres Amin aus Melamin, Anilin, Harnstoff und Formaldehyd hergestellt:
Man legte 216,52 g (2,08 Mol) Natriumhydrogensulfit in einem Dreihalskolben vor. Dann tropfte man 59,13 g (1,97 Mol) Formaldehyd zu. Das so entstandene Gemisch wurde auf 90 °C erhitzt und 10 Minuten bei 90°C gerührt. Dann gab man 37,30 g (0,40 mol) Anilin zu und rührte das Gemisch 15 Minuten bei 90 °C weiter. Man gab 15,00 g (0,12 Mol) Melamin und 25,30 g (0,42 mol) Harnstoff zu und tropfte 16,23 g (0,54 mol) Formaldehyd langsam zu. Die so entstandene Mischung wurde 4 Stunden bei 90 °C gerührt und das entstehende Anilinharzöl danach auf Raumtemperatur abgekühlt. Es hatte eine Aminzahl von 77,5 mg KOH/g, bestimmt nach DIN 53176.

50,0 g (0,039 Mol) Polyisobutenylbernsteinsäureanhydrid mit einer Verseifungszahl von 87,5 (Mᵣₑₐₖₜᵢᵥ = 1282 g/mol) wurden mit 8,6 g (0,0039 mol bezogen auf Anilin) Anilinharzöl versetzt und auf 130 °C erhitzt. Das so entstehende Reaktionsgemisch wurde 7 Stunden bei 130°C gerührt und danach auf Zimmertemperatur abgekühlt. Man erhielt 47,7 g erfindungsgemäßes Gemisch als homogen aussehendes, klares, hellbraunes Kondensat mit einer Viskosität von 277.000 mPa·s bei 23 °C.

### I.7 Herstellung von erfindungsgemäßem Gemisch I.7

In einer Rührapparatur wurden 125,0 g (0,469 mol) (*Z*)-Dodecenylbernsteinsäureanhydrid (IV.2) 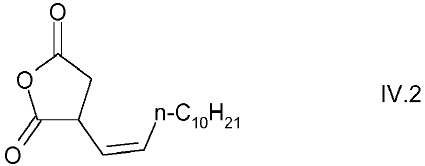 mit 19,73 g (0,156 mol) Melamin versetzt und auf 130 °C erhitzt. Das entstehende Reaktionsgemisch wurde 7 Stunden bei 130°C gerührt und danach auf Zimmertemperatur abgekühlt. Man erhielt 137,6 g erfindungsgemäßes Gemisch I.7 als homogenes, undurchsichtiges, hellbraunes, dickes Kondensat. Die dynamische Viskosität betrug 750 mPa·s bei 40 °C. Charakteristische Peaks im IR-Spektrum: 1785 cm⁻¹ und 1862 cm⁻¹, 1785 cm⁻¹ und 1712 cm⁻¹.

### I.8 Herstellung von erfindungsgemäßem Gemisch I.8

500,0 g (0,392 Mol) Polyisobutenylbernsteinsäureanhydrid mit einer Verseifungszahl von 87,5 mg KOH/g (Mᵣₑₐₖₜᵢᵥ = 1282 g/mol) wurden mit 16,4 g (0,130 mol) Melamin versetzt und auf 300 °C erhitzt. Das entstehende Reaktionsgemisch wurde 4 Stunden bei 300 °C gerührt und danach auf Zimmertemperatur abgekühlt. Man erhielt 434,5 g erfindungsgemäßes Gemisch I.8 als homogenes, dunkelbraunes, dickes und klebendes Kondensat. Die dynamische Viskosität betrug 31.800 mPas bei 40 °C. Charakteristische Peaks im IR-Spektrum: 1780 cm⁻¹ und 1716 cm⁻¹.

### I.9 Herstellung von erfindungsgemäßem Gemisch I.9

125,0 g (0,098 Mol) Polyisobutenylbernsteinsäureanhydrid mit einer Verseifungszahl von 87,5 mg KOH/g (Mᵣₑₐₖₜᵢᵥ = 1282 g/mol) wurden mit 4,1 g (0,033 mol) Melamin und 9,9 g (0,098 Mol) Triethylamin versetzt und auf 90 °C erhitzt. Das entstehende Reaktionsgemisch wurde 6 Stunden bei 90 °C gerührt und danach auf Zimmertemperatur abgekühlt. Nach Destillation des Triethylamins erhielt man 115,6 g erfindungsgemäßes Gemisch I.9 als homogenes, dunkelbraunes, dickes und klebendes Kondensat. Die dynamische Viskosität betrug 56.500 mPas bei 40 °C.
Charakteristische Peaks im IR-Spektrum: 1780 cm⁻¹ und 1716 cm⁻¹.

### I.10 Herstellung von erfindungsgemäßem Gemisch I.10

In einer Rührapparatur wurden 125,0 g (0,469 mol) Dodecenylbernsteinsäureanhydrid mit 19,73 g (0,156 Mol) Melamin versetzt und auf 300 °C erhitzt. Das entstehende Reaktionsgemisch wurde 4 Stunden bei 300 °C gerührt und danach auf Zimmertemperatur abgekühlt. Man erhielt 120,5 g erfindungsgemäßes Gemisch I.10 als trübes, hellbraunes, dickes Kondensat. Die dynamische Viskosität betrug 74.100 mPa·s bei 40 °C. Charakteristische Peaks im IR-Spektrum: 1778 cm⁻¹ und 1712 cm⁻¹.

### II. Herstellung von Leder

### II.1 Herstellung von Fettungsmitteln

In einem Rührgefäß wurden 2,3 g eines Polyisobutens (Mₙ: 1000 g/mol) mit 300 g n-C₁₈H₃₇O(CH₂CH₂O)₂₅H, 400 g Ölsäure und 2,3 kg sulfitiertes, oxidiertes Triolein gemischt und auf 60°C erwärmt. Danach gab man 4,7 l Wasser und 100 g n-C₁₈H₃₇O(CH₂CH₂O)₇H zu. Die entstandene Emulsion wurde durch einen Spalthomogenisator geleitet und auf Zimmertemperatur abgekühlt. Man erhielt Fettlicker FL-1.

Man vermischte jeweils in einem Becherglas mittels eines handelsüblichen Stabmixers der Fa. IKA jeweils 92 Gew.-% des Fettungsmittel FL-1 (s.u.) und jeweils 8 Gew.-% der erfindungsgemäßen Gemische I.1 bis I.6. Man erhielt Fettungsmittel F.1 bis F.6 gemäß Tabelle 1.

**Tabelle 1: Zusammensetzung von Fettungsmitteln F.1 bis F.6**

| Fettungsmittel | FL-1 [Gew.-%] | (Gemisch) [Gew.-%] |
|---|---|---|
| F.1 | 92 | 8 (I.1) |
| F.2 | 92 | 8 (I.2) |
| F.3 | 92 | 8 (I.3) |
| F.4 | 92 | 8 (I.4) |
| F.5 | 92 | 8 (I.5) |
| F.6 | 92 | 8 (I.6) |

### II.2 Nachgerbung von Leder

Angaben in Gew.-% bezeichnen den Wirkstoff und beziehen sich jeweils auf das Falzgewicht, wenn nicht anders angegeben.

100 Gewichtsteile Chrom-gegerbtes Rindleder der Falzstärke 1,8 bis 2,0 mm wurden in einem drehbaren Fass (50 I) mit strömungsbrechenden Einbauten bei 30 °C mit 200 Gew.-% Wasser 10 Minuten gewalkt. Anschließend wurde das Wasser abgelassen und das Rindleder mit 1 Gew.-% Natriumformiat und 1,5 Gew.-% eines Naphthalinsulfonsäure-Formaldehyd-Kondensationsprodukts, hergestellt nach US 5,186,846, Beispiel "Dispergiermittel 1", in 100 Gew.-% Wasser gewalkt (60 Minuten), wobei eine Neutralisation stattfand. Das so vorbehandelte Rindleder wurde dann im Kernbereich in 7 Streifen zu je ca. 500 g geschnitten. Die Nachgerbung erfolgte bis zum Differenzierungsschritt durch die Zugabe des jeweiligen erfindungsgemäßen Gemisches in einem Fass und wurde erst danach in sieben separaten 10-I-Dose-Fässern komplettiert.

Man hob den pH-Wert der Leder und Lederflotte in vier Schritten mit jeweils 1,5 Gew.-% NaHCO₃ auf 7 an. Anschließend wurden 10 Gew.-% Farbstoff aus WO 05/040490, Beispiel 17g (schwarz), zugesetzt und 40 Minuten bei einem pH-Wert von 7 gewalkt. Mit 8 Gew.-% Soda wurde der pH-Wert auf 9,6 angehoben und weitere 80 Minuten gewalkt. Sodann wurde zweimal mit je 300 Gew.-% Wasser gewaschen und das Waschwasser verworfen. In einer neuen Flotte, zusammengesetzt aus 150 Gew.-% Wasser und 1,5 Gew.-% Ameisensäure (pH-Wert 5,6), wurde 50 Minuten gewalkt.

Nach dem Ablassen der neuen Flotte wurden die Leder auf die sieben separaten 10-I-Dose-Fässer verteilt und zum Zweck der Nachgerbung zunächst jeweils nacheinander 80 Gew.-% Wasser und 2 Gew.-% eines Hydrophobiermittels nach WO 2004/072307, Beispiel 1, Formulierung 1.1 dosiert. Nach 10 Minuten versetzte man mit 6 Gew.-% Sulfongerbstoff aus EP-B 0 459 168, Beispiel K1, und walkte weitere 30 Minuten.

Die Fettung erfolgt durch die Zugabe von 14 Gew.-% Fettungsmittel gemäß Tabelle 1 und 2. In dem Vergleichsversuch erfolgte Behandlung mit 14% FL-1. Nach einer Walkzeit von 60 Minuten wurde ein pH-Wert von 3,5 durch portionsweises Dosieren von insgesamt 5 Gew.-% Ameisensäure eingestellt. Die Flotte wurde abgelassen, und es wurde abschließend mit 300 Gew.-% Wasser nachgewaschen. Man trocknete die so erhältlichen Leder bei 45 °C, 20 mbar über einen Zeitraum von 6 Minuten. Nach dem Stollen und Ruhen der Leder (4 h bei 20°C) prüfte man die erfindungsgemäßen Leder L1 bis L6 sowie das Vergleichsleder V-L7 (siehe Tabelle 2). Anschließend wurden die erfindungsgemäßen Leder L1 bis L6 sowie das Vergleichsleder V-L7 einem dreistufigen Maschinenwaschzyklus gemäß DIN EN ISO 15702 unterzogen und anschließend erneut bewertet (Tabelle 3).

**Tabelle 2: Anwendungstechnische Eigenschaften der erfindungsgemäßen Leder L1 bis L6 und Vergleichsleder V-L7**

| Beispiel | Fettungsmittel | Leder | Fülle | Weichheit | Färbung: Egalität | Färbung: Intensität, Brillanz | Festnarbigkeit | Auszehrung |
|---|---|---|---|---|---|---|---|---|
| I.1 | F.1 | L1 | 2,5 | 3 | 2,5 | 2,5 | 2 | 1,5 |
| I.2 | F.2 | L2 | 1,5 | 2 | 2 | 1,5 | 2 | 1,5 |
| I.3 | F.3 | L3 | 2 | 2,5 | 2 | 2 | 2,5 | 2 |
| I.4 | F.4 | L4 | 3 | 3 | 2,5 | 3 | 3 | 2 |
| I.5 | F.5 | L5 | 2,5 | 2 | 3 | 2,5 | 2,5 | 2 |
| I.6 | F.6 | L6 | 2 | 2,5 | 2,5 | 3 | 2 | 2 |
| V-I.7 | FL-1 | V-L7 | 3 | 3,5 | 2,5 | 3 | 2,5 | 3 |

### Anmerkungen:

Die Bewertung erfolgte nach einem Notensystem von 1 (sehr gut) bis 5 (mangelhaft).

**Tabelle 3: Anwendungstechnische Eigenschaften der erfindungsgemäßen Leder L1 bis L7 nach dem dreistufigen Waschzyklus gem. DIN EN ISO 15702**

| Fettungsmittel | Leder | Fülle | Weichheit | Färbung: Egalität | Färbung: Intensität u. Brillanz | Festnar-bigkeit | Fleischseite, Weichheit |
|---|---|---|---|---|---|---|---|
| F.1 | L1 | 2,5 | 3 | 2,5 | 2,5 | 2,5 | 1,5 |
| F.2 | L2 | 2 | 2 | 1,5 | 2 | 2 | 2 |
| F.3 | L3 | 2 | 2,5 | 2 | 2 | 2,5 | 2,5 |
| F.4 | L4 | 3 | 3 | 2,5 | 3 | 3 | 3 |
| F.5 | L5 | 3,5 | 2,5 | 3 | 3 | 3 | 2 |
| F.6 | L6 | 2,5 | 3 | 3 | 3 | 2,5 | 3 |
| FL-1 | V-L7 | 4 | 5 | 4 | 5 | 3,5 | 5 |

Es fällt auf, dass die erfindungsgemäßen Leder an sich weicher und runder sind als das Vergleichsleder. Noch deutlicher insbesondere hinsichtlich der Färbung fallen die Unterschiede nach dem dreistufigen Maschinenwaschzyklus aus. Das Vergleichsleder V-L7 kann nach Durchlaufen von einem dreistufigen Maschinenwaschzyklus nicht mehr als gefälliges Leder bezeichnet werden.

## Patentansprüche

1. Verfahren zur Herstellung von Leder unter Verwendung von einem oder mehreren Umsetzungsprodukten von
(a) Melamin
(b) mit mindestens einer Verbindung der Formel I 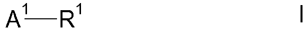
wobei R¹ gewählt wird aus Kohlenwasserstoffresten mit 10 bis 5000 C-Atomen, unverzweigt oder verzweigt, gesättigt oder mit einer bis zu drei C-C-Doppelbindungen,
und A¹ aus mit Aminen zur Reaktion befähigten Gruppen.

2. Verfahren nach Anspruch 1, **dadurch gekennzeichnet, dass** es sich bei R¹ um einen Kohlenwasserstoffrest der Formel 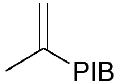 oder um einen n-C₁₂-C₄₀-Alkylrest handelt, wobei PIB einen Rest bezeichnet, der von Polyisobuten abgeleitet ist.

3. Verfahren nach Anspruch 1 oder 2, **dadurch gekennzeichnet, dass** A¹ gewählt wird aus Säurechloridgruppen, C₁-C₄-Alkylestergruppen und Carbonsäureanhydridgruppen.

4. Verfahren nach einem der Ansprüche 1 bis 3, **dadurch gekennzeichnet, dass** man zusätzlich mit mindestens einer hydrophoben Verbindung behandelt, gewählt aus hydrophoben Silikonen, Polyisobuten und nativen Fetten.

5. Leder, hergestellt nach einem der Ansprüche 1 bis 4.

6. Bekleidungsstücke, hergestellt unter Verwendung von Leder nach Anspruch 5.

7. Autoinnenteile, hergestellt unter Verwendung von Leder nach Anspruch 5.

8. Verbindungen der Formel II 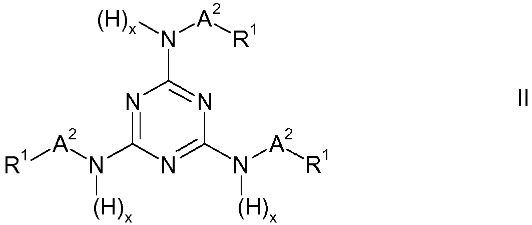 in denen die Variablen wie folgt definiert sind:
R¹ gleich oder verschieden und gewählt aus Kohlenwasserstoffresten mit 10 bis 5000 C-Atomen, unverzweigt oder verzweigt, gesättigt oder mit einer bis zu drei C-C-Doppelbindungen
N-A² gleich oder verschieden und gewählt aus funktionellen Einheiten, die durch Reaktion von mit Amin zur Reaktion befähigten Gruppen A¹ bei der Reaktion mit Amin entstehen,
x gleich oder verschieden und jeweils gewählt aus Null oder eins.

9. Verbindungen der Formel III a oder III b, 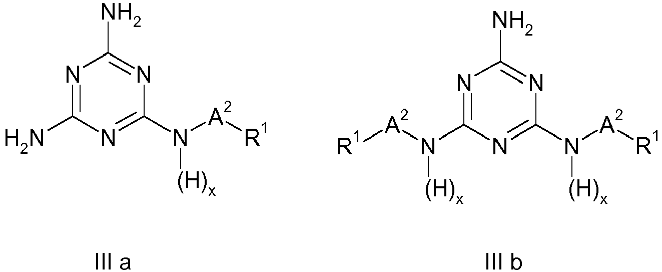 in denen die Variablen wie folgt definiert sind:
R¹ gleich oder verschieden und gewählt aus Kohlenwasserstoffresten mit 10 bis 5000 C-Atomen, unverzweigt oder verzweigt, gesättigt oder mit einer bis zu drei C-C-Doppelbindungen
N-A² gleich oder verschieden und gewählt aus funktionellen Einheiten, die durch Reaktion von mit Amin zur Reaktion befähigten Gruppen A¹ bei der Reaktion mit Amin entstehen,
x gleich oder verschieden und jeweils gewählt aus Null oder eins.

10. Verbindungen nach Anspruch 8 oder 9, **dadurch gekennzeichnet, dass** N-A² gewählt wird aus Gruppen der Formel 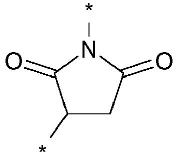 und dass x jeweils gleich null ist.

11. Gemische, enthaltend mindestens eine Verbindung nach einem der Ansprüche 8 bis 10 sowie Polyisobuten.

12. Gemische nach Anspruch 11, **dadurch gekennzeichnet, dass** mindestens eine Verbindung nach einem der Ansprüche 8 bis 10 und Polyisobuten in einem Gewichtsverhältnis im Bereich von 1 zu 10 bis 2 zu 1 vorliegen.

13. Verwendung von Gemischen nach einem der Ansprüche 11 oder 12 als Hilfsmittel zur Lederherstellung.

14. Verfahren zur Herstellung von Leder unter Verwendung von Gemischen nach Anspruch 11 oder 12.

15. Verfahren nach einem der Ansprüche 1 bis 4 oder 14, **dadurch gekennzeichnet, dass** man es als Gerbverfahren oder als Nachgerbverfahren ausübt.

16. Verfahren zur Herstellung von Verbindungen nach einem der Ansprüche 8 bis 10, **dadurch gekennzeichnet, dass** man
(a) Melamin
umsetzt mit
(b) mindestens einer Verbindung der allgemeinen Formel I 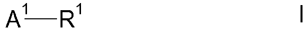
wobei R¹ gewählt wird aus Kohlenwasserstoffresten mit 10 bis 5000 C-Atomen, unverzweigt oder verzweigt, gesättigt oder mit einer bis zu drei C-C-Doppelbindungen,
und A¹ aus mit Aminen zur Reaktion befähigten Gruppen.

17. Verfahren nach Anspruch 16, **dadurch gekennzeichnet, dass** man Melamin umsetzt mit einer Verbindung der allgemeinen Formel IV 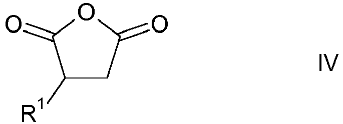 wobei R¹ gewählt wird aus Kohlenwasserstoffresten mit 10 bis 5000 C-Atomen, unverzweigt oder verzweigt, gesättigt oder mit einer bis zu drei C-C-Doppelbindungen.

## Claims

1. Method for the production of leather using one or more reaction products of
(a) melamine
(b) with at least one compound of formula I 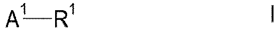
wherein R¹ is selected from hydrocarbon radicals with 10 to 5000 C atoms, unbranched or branched, saturated or with one to three C-C double bonds,
and A¹ is selected from groups capable of reacting with amines.

2. Method according to claim 1, **characterised in that** R¹ is a hydrocarbon radical of the formula 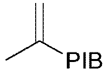 or an n-C₁₂-C₄₀-alkyl radical, wherein PIB denotes a radical which is derived from polyisobutene.

3. Method according to claim 1 or 2, **characterised in that** A¹ is selected from acid chloride groups, C₁-C₄-alkyl ester groups and carboxylic acid anhydride groups.

4. Method according to any one of claims 1 to 3, **characterised in that** one additionally treats with at least one hydrophobic compound, selected from hydrophobic silicones, polyisobutene and natural fats.

5. Leather, produced according to any one of claims 1 to 4.

6. Items of clothing, produced using leather according to claim 5.

7. Car interior parts, produced using leather according to claim 5.

8. Compounds of formula II 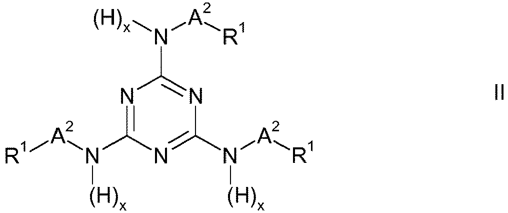 in which the variables are defined as follows:
R¹ are the same or different and selected from hydrocarbon radicals with 10 to 5000 C atoms, unbranched or branched, saturated or with one to three C-C double bonds
N-A² are the same or different and selected from functional units which result from the reaction with amine through reaction of groups of A¹ capable of reacting with amine,
x are the same or different and selected from zero or one, respectively.

9. Compounds of formula III a or III b, 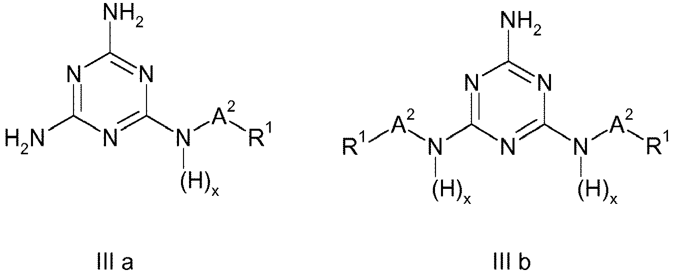 in which the variables are defined as follows:
R¹ are the same or different and selected from hydrocarbon radicals with 10 to 5000 C atoms, unbranched or branched, saturated or with one to three C-C double bonds
N-A² are the same or different and selected from functional units which result from the reaction with amine through reaction of groups of A¹ capable of reacting with amine,
x are the same or different and selected from zero or one, respectively.

10. Compounds according to claim 8 or 9, **characterised in that** N-A² is selected from groups of the formula 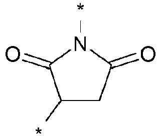 and that x is equal to zero, respectively.

11. Mixtures, containing at least one compound according to any one of claims 8 to 10, as well as polyisobutene.

12. Mixtures according to claim 11, **characterised in that** at least one compound according to any one of claims 8 to 10 and polyisobutene are present in a weight ratio ranging from 1 to 10 to 2 to 1.

13. Use of mixtures according to any one of claims 11 or 12 as auxiliary material for leather production.

14. Method for the production of leather using mixtures according to claim 11 or 12.

15. Method according to any one of claims 1 to 4 or 14, **characterised in that** it is performed as a tanning method or a post-tanning method.

16. Method for the production of compounds according to any one of claims 8 to 10, **characterised in that**
(a) melamine
is reacted with
(b) at least one compound of general formula I 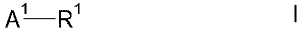
wherein R¹ is selected from hydrocarbon radicals with 10 to 5000 C atoms, unbranched or branched, saturated or with one to three C-C double bonds,
and A¹ is selected from groups capable of reacting with amines.

17. Method according to claim 16, **characterised in that** melamine is reacted with a compound of general formula IV 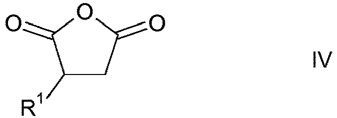 wherein R¹ is selected from hydrocarbon radicals with 10 to 5000 C atoms, unbranched or branched, saturated or with one to three C-C- double bonds.

## Revendications

1. Procédé de fabrication de cuir par utilisation d'un ou plusieurs produits de réaction
(a) de la mélamine
(b) avec au moins un composé de formule I 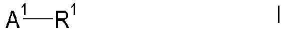
dans laquelle R¹ est choisi parmi les résidus hydrocarbonés ayant 10 à 5000 atomes de carbone, non ramifiés ou ramifiés, saturés ou comportant une à trois doubles liaisons C-C,
et A¹ est choisi parmi les groupes pouvant réagir avec les amines.

2. Procédé selon la revendication 1, **caractérisé en ce que**, pour ce qui concerne R¹, il s'agit d'un résidu hydrocarboné de formule 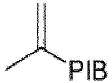 ou d'un résidu n-alkyle en C₁₂-C₄₀, PIB désignant un résidu qui dérive du polyisobutène.

3. Procédé selon la revendication 1 ou 2, **caractérisé en ce que** A¹ est choisi parmi les groupes chlorure d'acyle, les groupes ester alkylique en C₁-C₄ et les groupes anhydride carboxylique.

4. Procédé selon l'une des revendications 1 à 3, **caractérisé en ce qu'**on procède en outre à un traitement avec au moins un composé hydrophobe, choisi parmi les silicones hydrophobes, le polyisobutène et les graisses natives.

5. Cuir fabriqué conformément à l'une des revendications 1 à 4.

6. Pièces de vêtement, fabriquées par utilisation du cuir selon la revendication 5.

7. Pièces intérieures pour automobiles, fabriquées par utilisation du cuir selon la revendication 5.

8. Composés de formule II 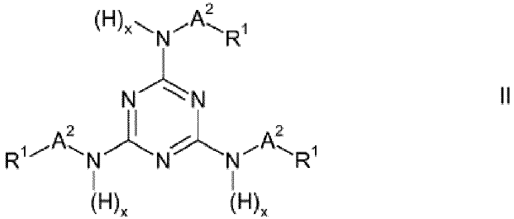 dans laquelle les variables sont définies comme suit :
les radicaux R¹ sont identiques ou différents et sont choisis parmi les résidus hydrocarbonés ayant 10 à 5000 atomes de carbone, non ramifiés ou ramifiés, saturés, ou comportant une à trois doubles liaisons C-C,
les fragments N-A² sont identiques ou différents et sont choisis parmi les unités fonctionnelles qui se forment par réaction de groupes A¹ pouvant réagir avec une amine, lors de la réaction avec l'amine,
les indices x sont identiques ou différents et sont chacun choisis parmi zéro ou un.

9. Composés de formule IIIa ou de formule IIIb, 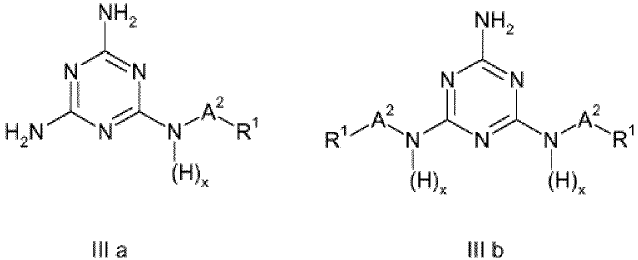 dans lesquelles les variables sont définies comme suit :
les radicaux R¹ sont identiques ou différents et sont choisis parmi les résidus hydrocarbonés ayant 10 à 5000 atomes de carbone, non ramifiés ou ramifiés, saturés, ou comportant une à trois doubles liaisons C-C,
les fragments N-A² sont identiques ou différents et sont choisis parmi les unités fonctionnelles qui se forment par réaction de groupes A¹ pouvant réagir avec une amine, lors de la réaction avec l'amine,
les indices x sont identiques ou différents et sont chacun choisis parmi zéro ou un.

10. Composés selon la revendication 8 ou 9, **caractérisés en ce que** N-A² est choisi parmi les groupes de formule 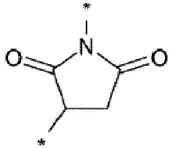 et **en ce que** chaque x vaut zéro.

11. Mélanges contenant au moins un composé selon l'une des revendications 8 à 10, ainsi que du polyisobutène.

12. Mélanges selon la revendication 11, **caractérisés en ce qu'**au moins un composé selon l'une des revendications 8 à 10 et du polyisobutène sont présents selon un rapport en poids compris dans la plage de 1:10 à 2:1.

13. Utilisation de mélanges selon l'une des revendications 11 à 12 en tant qu'adjuvant pour la fabrication du cuir.

14. Procédé de fabrication de cuir par utilisation de mélanges selon la revendication 11 ou 12.

15. Procédé selon l'une des revendications 1 à 4 ou 14, **caractérisé en ce qu'**on le met en oeuvre en tant que procédé de tannage ou procédé de retannage.

16. Procédé de préparation de composés selon l'une des revendications 8 à 10, **caractérisé en ce qu'**on fait réagir
(a) de la mélamine
(b) avec au moins un composé de formule générale I 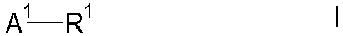
dans laquelle R¹ est choisi parmi les résidus hydrocarbonés ayant 10 à 5000 atomes de carbone, non ramifiés ou ramifiés, saturés ou comportant une à trois doubles liaisons C-C,
et A¹ est choisi parmi les groupes pouvant réagir avec les amines.

17. Procédé selon la revendication 16, **caractérisé en ce qu'**on fait réagir de la mélamine avec un composé de formule générale IV 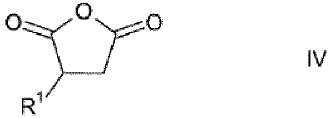 dans laquelle R¹ est choisi parmi les résidus hydrocarbonés ayant 10 à 5000 atomes de carbone, non ramifiés ou ramifiés, saturés, ou comportant un à trois doubles liaisons C-C.
